(19) Europäisches Patentamt
European Patent Office
Office européen des brevets

(11) Numéro de publication: **0 291 389 B1**

(12) # FASCICULE DE BREVET EUROPEEN

(45) Date de publication de fascicule du brevet: **09.12.92**

(51) Int. Cl.5: **C08J 3/12**, C08J 3/02, G01N 33/545, G01N 33/546, G01N 33/547

(21) Numéro de dépôt: **88401078.6**

(22) Date de dépôt: **04.05.88**

Le dossier contient des informations techniques présentées postérieurement au dépôt de la demande et ne figurant pas dans le présent fascicule.

(54) **Particules de polymère comportant, implantées à leur surface, des molécules amphiphiles portant des groupes ionogènes ou réactifs, leur procédé de préparation et leur application en biologie.**

(30) Priorité: **11.05.87 FR 8706549**

(43) Date de publication de la demande:
**17.11.88 Bulletin 88/46**

(45) Mention de la délivrance du brevet:
**09.12.92 Bulletin 92/50**

(84) Etats contractants désignés:
**AT BE CH DE ES FR GB GR IT LI LU NL SE**

(56) Documents cités:
**EP-A- 0 187 391**
**DE-A- 2 255 622**
**GB-A- 1 503 547**
**US-A- 4 115 535**

**CHEMICAL ABSTRACTS, vol. 107, no. 20, 16 novembre 1987, page 492, résumé no. 183538b, Columbus, Ohio, US; S. WINTERS: "Immobilized heparin via a long chain poly(ethylene oxide) spacer for protein and platelet compatibility"**

(73) Titulaire: **RHONE-POULENC CHIMIE**
**25, quai Paul Doumer**
**F-92408 Courbevoie Cédex(FR)**

(72) Inventeur: **Chauvel, Bernard**
**24, Chemin de la Commanderie**
**F-95120 Ermont(FR)**
Inventeur: **Daniel, Jean-Claude**
**Résidence "Le Panorama" 13, rue de Neuilly**
**F-94120 Fontenay/Sous/Bois(FR)**
Inventeur: **Pusineri, Christian**
**33, rue des Fleurs Serezin du Rhône**
**F-69360 Saint Symphorien d'Ozon(FR)**

(74) Mandataire: **Seugnet, Jean Louis et al**
**RHONE-POULENC CHIMIE Direction de la Propriété Industrielle 25, Ouai Paul Doumer**
**F-92408 Courbevoie Cedex(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

## Description

La présente invention a pour objet des particules de polymère comportant, implantées à leur surface, des molécules amphiphiles portant des groupes ionogènes ou réactifs, lesdites particules se présentant telles quelles ou en dispersion aqueuse, leur procédé de préparation et leur application en biologie.

Le but de la Demanderesse a été de trouver des particules de polymère portant des motifs ionogènes ou réactifs suffisamment éloignés de la surface desdites particules pour pouvoir fixer, sur lesdits motifs, des molécules encombrantes telles que les protéines et ce sans risque de modifier l'activité desdites molécules par contact avec la surface macromoléculaire.

Le brevet GB-A-1 503 547 décrit des réactifs latex pour réactions sérologiques qui comprennent des particules de polymères sur lesquelles est adsorbé un tensio-actif non-ionique d'un polymère d'oxyde d'éthylène et qui ont été sensibilisés avec une substance sérologiquement active. Il s'avère, lorsque l'on suit le mode d'adsorption du tensio-actif non-ionique sur les particules décrit dans ce document, que la quantité adsorbée de tensio-actif est faible et insuffisante.

Selon l'invention, lesdites particules de polymère sont caractérisées en ce qu'elles comportent, implantées à leur surface, des molécules d'un composé amphiphile de HLB supérieur ou égal à 10 et de masse moléculaire supérieure ou égale à 400 constitué d'une séquence hydrophile oligomère terminée par au moins un groupe ionogène ou réactif et d'une séquence hydrophobe, la quantité de molécules amphiphiles implantées correspondant à $10^{-5}$ à $10^{-1}$ mole, de préférence de $10^{-4}$ à $10^{-2}$ mole pour 100 g de polymère, le polymère constituant lesdites particules présentant une température de transition vitreuse Tg supérieure à environ 40 ° C, et de préférence supérieure à environ 70 ° C, le diamètre desdites particules étant de l'ordre de 0,01 à 50 $\mu$m et l'implantation étant telle que le composé amphiphile pénètre par sa partie hydrophobe dans la couche périphérique de la particule et que ladite partie hydrophobe est emmêlée avec les chaînes de polymère, le composé amphiphile n'étant ainsi pas simplement adsorbé à la surface des particules.

Lesdites particules présentent un diamètre de préférence de l'ordre de 0,1 à 15 $\mu$m et généralement de l'ordre de 0,3 à 5 $\mu$m.

Parmi les polymères pouvant constituer lesdites particules on peut citer les homopolymères ou les copolymères contenant des motifs dérivés :

- des monomères vinylaromatiques (styrène, vinyltoluène)
- des alkylesters d'acides $\alpha$-$\beta$ insaturés (acrylates et méthacrylates de méthyle, éthyle)
- des esters d'acides carboxyliques insaturés (acétate de vinyle)
- du chlorure de vinyle ; du chlorure de vinylidène
- des diènes (butadiène)
- de ceux présentant des fonctions nitriles (acrylonitrile)

Les composés amphiphiles implantés en surface présentent de préférence un HLB supérieur ou égal à 10 et inférieur à 20. Ils présentent une séquence hydrophile oligomère comme par exemple une séquence polyoxyalkylénée contenant de 5 à 100, de préférence de 5 à 50, motifs oxyalkylénés en $C_2$-$C_3$, une séquence polycarboxyethylénée et/ou polyamidoéthylénée et/ou polycyanoéthylénée contenant de 4 à 50 motifs carboxyéthylénés et /ou amidoéthylénés et/ou cyanoéthylénés, ladite séquence hydrophile oligomère étant terminée par un groupe ionogène ou réactif.

Parmi les terminaisons ionogènes ou réactives, on peut citer les groupements :

-OH, $-SO_3H$, -COOH, -CHO, $-ØCH_2Cl$, $-NH_2$, $-NR_2$, $NR_3$ (R étant un radical alkyle en $C_1$ - $C_2$), $-CONH_2$, $-NH-NH_2$, $-NH-NH-CO-NH_2$, -SH,

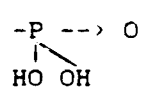

A titre d'exemple de composé amphiphile on peut citer :

- les alcools gras ou les acides gras polyoxyéthylénés et/ou polyoxypropylénés présentant de 5 à 50 motifs oxyalkylènes et dont la séquence hydrophobe contient environ de 8 à 20 atomes de carbone (tels que les CEMULSOL DB commercialisés par la Société Française d'Organo Synthèse, les SOPROPHOR LA commercialisés par Rhône-Poulenc)
- les esters desdits alcools gras portant des fonctions semicarbazides (tels que les esters desdits alcools et de l'acide phenylcarbamique meta-semicarbazide para-méthyle), des fonctions acides (tels que les monoesters desdits alcools et de l'acide azélaïque), des fonctions thiols (tels que les esters

desdits alcools avec l'acide thioglycolique)

- les amides desdits acides gras (tels que les ETHOMID commercialisés par Armour Industrial Chemical Co)
- les alkylphénols polyoxyalkylénés et/ou polyoxypropylénés présentant de 5 à 50 motifs oxyalkylènes et dont le ou les radicaux alkyles contiennent de 8 à 12 atomes de carbone, et leurs esters avec l'acide phosphorique (tels que les SOPROPHOR BC et OP commercialisés par Rhône-Poulenc, les SOPROPHOR NFP commercialisés par Geronazzo, les GAFAC RE commercialisés par General Aniline and Film Corp.)
- les amines grasses polyoxyéthylénées dont la séquence hydrophobe contient de 8 à 22 atomes de carbone (telles que les SOPROMINE commercialisés par Rhône-Poulenc, les ETHOMEEN commercialisés par Armour Industrial Co)

Les particules faisant l'objet de l'invention peuvent correspondre à une certaine distribution ou bien être calibrées.

Une variante desdites particules consiste en ce qu'elles sont magnétisables.

Lesdites particules magnétisables contiennent de 0,5 à 50 % en poids (de préférence de 0,5 à 35 % et tout particulièrement de 0,7 à 20 %) d'une charge magnétique dont la taille est inférieure à 1 $\mu$m et de préférence comprise entre 0,002 - 0,05 $\mu$m ; la charge magnétique est bien évidemment suffisamment fine pour pouvoir être incluse dans les particules de polymère.

Cette charge magnétique peut être constituée par exemple par :

. des métaux ou leurs alliages tels que : fer, fer-silicium, nickel, cobalt, ou leurs alliages avec du molybdène, du chrome, du cuivre, du vanadium, du manganèse, de l'aluminium,du titane;

. des oxydes de fer : $Fe_3O_4$ ou $\gamma$-$Fe_2O_3$ pur ou en combinaison ou en mélange avec d'autres oxydes comme les oxydes de cobalt, manganèse, zinc, baryum, terres rares ;

. du dioxyde de chrome.

Les particules faisant l'objet de l'invention peuvent être obtenues selon un procédé caractérisé en ce qu'il comprend les étapes suivantes :

1) ledit composé amphiphile de HLB supérieur ou égal à 10 et de masse moléculaire supérieure ou égale à 400 est mis en contact avec un latex de particules dudit polymère dont la température de transition vitreuse Tg est supérieure à environ 40 °C, lesdits particules constituant le latex présentant un diamètre de l'ordre de 0,01 à 50 $\mu$m, la quantité dudit composé amphiphile mis en oeuvre correspondant à $10^{-4}$ à 1 mole, de préférence à $10^{-3}$ à $10^{-1}$ mole,pour 100 g de polymère, cette opération de mise en contact étant réalisée à une température comprise dans la zone de transition vitreuse dudit polymère, jusqu'à emmêlement des chaines de polymère et des séquences hydrophobes dudit composé amphiphile

2) élimination de tout composé amphiphile non fixé par implantation

3) séparation des particules ainsi obtenues.

Ces composés amphiphiles pouvant être mis en oeuvre sont ceux déjà cités ci-dessus.

Les latex pouvant être mis en oeuvre comportent environ de 1 à 50 %, de préférence 5 à 30 % et tout particulièrement de 10 à 15 %, en poids de particules de polymère, dont la nature a déjà été indiquée ci-dessus, lesdites particules présentant un diamètre de préférence de l'ordre de 0,01 à 15 $\mu$m et généralement de l'ordre de 0,05 à 3 $\mu$m ; lesdites particules peuvent correspondre à une certaine distribution ou bien être calibrées.

Lorsque des particules magnétisables sont désirées, les latex de particules magnétisables peuvent être obtenus selon les procédés décrits dans le brevet européen n° 38.730 et le brevet américain n° 4.157.323.

La mise en contact du composé amphiphile et du latex est réalisée en introduisant le composé amphiphile éventuellement en solution aqueuse dans la phase aqueuse dudit latex, par exemple à température ordinaire, puis en élevant la température jusqu'à une température comprise dans la zone de transition vitreuse du polymère constituant le latex. Dans cette zone de température les chaines macromoléculaires acquièrent une certaine mobilité leur permettant de s'emmêler avec la partie hydrophobe du composé amphiphile et de fixer ledit composé à la surface des particules de latex. Cette opération dure environ 15 minutes à 4 heures et généralement de 1 à 3 heures.

Pour un latex de polystyrène par exemple, cette opération d'emmêlement est réalisée à une température de l'ordre de 70 à 80 °C.

Cette zone de température peut être abaissée si nécessaire en utilisant en outre des plastifiants tels que les phtalates de dialkyle, les hydrocarbures aliphatiques ou aromatiques contenant au moins 5 atomes de carbone.

Un autre moyen de diminuer cette zone de température consiste à réaliser l'opération de mise en contact en présence d'un solvant du composé amphiphile et gonflant des particules de polymère ; le

solvant facilite la pénétration du composé amphiphile par sa partie hydrophobe dans la couche périphérique de la particule de polymère où elle s'emmêle avec les chaines de polymère. Le solvant peut ensuite être éliminé par exemple par évaporation sous vide.

Parmi les solvants pouvant favorablement être mis en oeuvre, on peut citer les dérivés aromatiques (toluène, éthylbenzène, xylènes), les dérivés aromatiques chlorés (mono, di et trichlorobenzène), les hydrocarbures aliphatiques et cycliques (heptane, décane, cyclohexane, décaline), les dialkyl ethers, les alcools (pentanon, cyclohexanol), les esters (propionate de methyle).

Le composé amphiphile non implanté durant l'opération de mise en contact ainsi que tout constituant émulsifiant préalablement présent dans le latex sont ensuite éliminés par lavage aqueux, par exemple par ultrafiltration.

Les particules de polymère comportant le composé amphiphile implanté en surface ainsi obtenues peuvent être séparées du milieu aqueux par les méthodes classiques de sédimentation par centrifugation.

La présente invention a également pour objet des dispersions aqueuses ou latex desdites particules de polymère comportant, implantées à leur surface, des molécules de composé amphiphile, le taux d'extrait sec desdites dispersions allant de 1 à 50 %, de préférence de 5 à 30 et tout particulièrement de 10 à 15 % en poids.

Lesdits latex peuvent être préparés selon les méthodes connues par dispersion directe dans l'eau desdites particules.

Un mode préférentiel de réalisation desdits latex consiste à supprimer la troisième étape (séparation des particules du milieu de mise en contact après élimination de tout composé amphiphile non implanté) du procédé de préparation des particules de polymère décrit ci-dessus.

Les particules ou les latex de particules faisant l'objet de l'invention sont particulièrement intéressants en biologie, pour la fixation de molécules biologiques (anticorps, antigènes) par covalence.

La fixation des molécules biologiques par covalence sur les particules de polymères peut être réalisée par réaction de couplage, réaction faisant intervenir les groupements terminaux de la molécule amphiphile implantée et les groupements fonctionnels de la molécule biologique à fixer.

Cette réaction de couplage peut être réalisée selon des méthodes bien connues, par exemple :

. en faisant appel à des agents de couplage (tels que glutaraldéhyde, carbodiimide hydrosoluble)
. par activation des fonctions du polymère (par exemple par diazotation, par action du bromure de cyanogène, de l'hydrazine) puis réaction avec la molécule à fixer.

Les produits peuvent donc être utilisés pour la réalisation de tests de diagnostic du type agglutination, radioimmunologique, enzymatique.

Les exemples suivants sont donnés à titre indicatif.

## EXEMPLE 1

- Implantation de CEMULSOL DB 25/18, (en abréviation CA-OH : composé amphiphile à fonction alcool), comme agent amphiphile, sur les particules d'un latex de polystyrène calibré.

Formule moyenne du composé amphiphile :

$$C_{18}H_{37}\!\left(O\text{-}CH_2\text{-}CH_2\right)_{50}OH$$

On prépare un solution à 2 % en poids dans l'eau d'agent amphiphile.

50 g de cette solution, ce qui correspond à 0,535 mole d'agent amphiphile sont mélangés à 10 g de particules d'un latex de polystyrène calibré de 0,9 $\mu$m de diamètre moyen et de 33 % en poids d'extrait sec.

Le mélange est maintenu sous agitation pendant 2 heures à 70 °C.

Après refroidissement le latex obtenu est lavé par ultrafiltration à l'aide de 2,5 l d'eau épurée pour 10 g de particules de latex, pour éliminer les molécules de composé amphiphile non implanté ainsi que les émulsifiants présents dans le latex de départ.

Le latex reste stable après ultrafiltration, ce qui est bien un indice de l'implantation du composé amphiphile.

Le latex obtenu est dosé par résonnance magnétique nucléaire.

On constate que la quantité en poids d'agent amphiphile implanté dans 100 g de particules finales est de 6,4 (théorie = 9,1).

Le résultat figure au tableau I.

## EXEMPLE 2

On répète l'opération décrite à l'exemple 1 dans les mêmes conditions, en remplaçant les 10 g de CA-OH par respectivement 5 g et 20 g de CA-OH. Le résultat du dosage par RMN figure au tableau I.

EXEMPLE 4

- Implantation de CEMULSOL DB 25/18 comme agent amphiphile sur les particules d'un latex de polystyrène magnétisable.

On répète l'opération à l'exemple 2 en remplaçant le latex de polystyrène calibré mis en oeuvre par 10 g de particules d'un latex de polystyrène présentant un extrait sec de 33 %, constitué de particules de diamètre allant de 0,5 à 0,9 µm, avec un diamètre moyen de 0,7 µm et contenant 41,5 % en poids de γ $Fe_2O_3$ par rapport au poids de polymère. Le mélange est traité comme à l'exemple 1.

Le résultat du dosage par conductrimétrie figure au tableau I.

EXEMPLE 5

Implantation de CEMULSOL DB 25/18 comme agent amphiphile sur les particules d'un latex de polychlorure de vinyle.

On répète l'opération décrite à l'exemple 1 en remplaçant le latex de polystyrène calibré mis en oeuvre, par 10 g de particules d'un latex calibré de polychlorure de vinyle présentant un extrait sec de 37 % en poids et constitué de particules calibrées de 0,7 µm de diamètre moyen.

Le mélange est maintenu sous agitation pendant 2 heures à 80 °C. Après refroidissement, le latex obtenu est ultrafiltré.

Le résultat du dosage par conductrimétrie figure au tableau I.

EXEMPLE 6

Implantation de CEMULSOL DB 25/18 comme agent amphiphile sur les particules d'un latex de copolymère styrène/méthacrylate de méthyle 40/60 en poids.

On répète l'opération décrite à l'exemple 1 en remplaçant le latex de polystyrène calibré mis en oeuvre, par 10 g de particules d'un latex calibré de styrène/méthacrylate de méthyle 40/60 présentant un taux d'extrait sec de 30 % en poids et constitué de particules calibrées de 0,8 µm de diamètre moyen.

Le mélange est maintenu sous agitation pendant 2 heures à 90 °C.

Après refroidissement, le latex obtenu est ultrafiltré.

Le résultat du dosage par RMN figure au tableau I.

EXEMPLE 7

Implantation de l'ester de CEMULSOL DB 25/18 et de l'acide phénylcarbamique méta-semicarbazide para-méthyle, (en abréviation CASC : composé amphiphile à fonction semi-carbazide) comme agent amphiphile, sur les particules d'un latex calibré de polystyrène.

Formule moyenne du composé amphiphile :

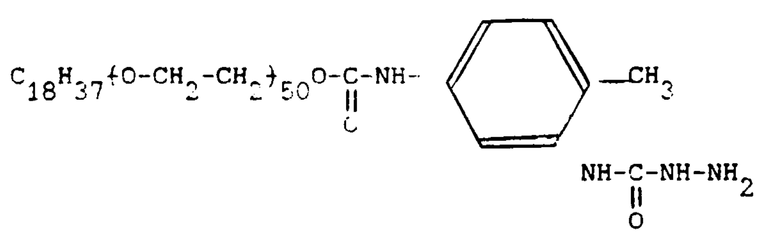

Préparation du composé amphiphile

Le composé est préparé en deux étapes :

- une première étape consistant à fonctionnaliser le CEMULSOL DB 25/18 par du toluène diisocyanate en présence de dioxanne comme solvant, à une température de 90 °C
- une deuxième étape consistant à fonctionnaliser le CEMULSOL DB 25/18 ainsi obtenu par addition

d'hydrate d'hydrazine en présence de dioxanne, à température ordinaire.

Le composé amphiphile recherché est précipité à froid dans de l'éther éthylique.

Implantation

On répète l'opération décrite à l'exemple 1 en remplaçant le CEMULSOL DB 25/18 par 50 g d'une solution aqueuse à 2 % de son dérivé préparé comme ci-dessus indiqué, ce qui correspond à 0,494 mmole de composé amphiphile.

Le résultat du dosage par RMN figure au tableau II.

EXEMPLE 8

Implantation de l'ester de CEMULSOL DB 25/18 et de l'acide thioglycolique (en abréviation CA-SH : composé amphiphile à fonction thiol) comme agent amphiphile, sur les particules d'un latex calibré de polystyrène.

Formule moyenne du composé amphiphile

$$C_{18}H_{37}\!\left(O-CH_2-CH_2\right)_{50}O-\underset{\underset{O}{\|}}{C}-CH_2-SH$$

Ce composé peut être préparé selon le procédé décrit dansle brevet américain n° 2.461.920.

On répète l'opération décrite à l'exemple 1 en remplaçant le CEMULSOL DB 25/18 par 50 g d'une solution aqueuse à 2 % de son ester thioglycolique de formule ci-dessus, ce qui correspond à 0,255 mmole de composé amphiphile.

Le résultat du dosage par conductimétrie figure au tableau II.

EXEMPLE 9

Implantation de l'ester de CEMULSOL DB 25/18 et de l'acide phénylcarbamique méta-semicarbazide para-méthyle, (en abréviation CASC), sur les particules d'un latex de polystyrène magnétisable.

On répète l'opération décrite à l'exemple 1 en remplaçant le latex calibré de polystyrène par 10 g d'un latex de polystyrène présentant un extrait sec de 33 % constitué de particules de diamètre allant de 0,5 à 0,9 µm avec un diamètre moyen de 0,7 µm et contenant 41,5 % en poids de $\gamma$ $Fe_2O_3$ par rapport au poids de polymère.

Le mélange est traité comme à l'exemple 1.

Le résultat du dosage par conductrimétrie figure au tableau II.

EXEMPLE 10

Implantation du monoester de CEMULSOL DB 25/18 et de l'acide azélaïque, (en abréviation CA-COOH : composé amphiphile à fonction acide), comme composé amphiphile, sur les particules d'un latex calibré de polystyrène .

Formule moyenne du composé amphiphile :

$$C_{18}H_{37}\!\left(O-CH_2-CH_2\right)_{50}O-\underset{\underset{O}{\|}}{C}-(CH_2)_7-COOH$$

Celui-ci peut être préparé par estérification du CEMULSOL DB 25/18 par l'acide azélaïque dans la pyridine en présence de dicyclohexylcarbodiimide comme agent de condensation.

On répète l'opération décrite à l'exemple 1 en remplaçant le CEMULSOL DB 25/18 par 50 g d'une solution à 2 % de son ester de formule ci-dessus, ce qui correspond à 0,495 mmole de composé amphiphile.

Le résultat du dosage par RMN figure au tableau II.

EXEMPLE 11

Implantation de l'ester de CEMULSOL DB 25/18 et de l'acide phénylcarbamique méta-semicarbazide para-méthyle (CASC), sur un latex calibré de polystyrène en présence d'un solvant gonflant.

On prépare 25 g d'une solution à 2 % en poids dans le toluène du composé amphiphile.

On prépare 10 g une solution à 0,1 % en poids dans l'eau de laurate d'ammonium, que l'on ajoute à 10 g de particules d'un latex de polystyrène présentant un taux d'extrait de 33 % en poids et constitué de particules calibrées de 0,8 μm de diamètre. La solution toluènique d'agent amphiphile est introduit dans le latex. Le mélange est maintenu pendant 2 heures à 45 ° C sous agitation.

Le toluène est chassé par stripping à 70 ° C sous une pression de 160mm Hg dans un évaporateur rotatif.

Le latex est ensuite lavé par ultrafiltration.

Le résultat du dosage par RMN figure au tableau II.

EXEMPLE 12

Les particules de latex obtenues à l'exemple I sont sédimentées à l'aide d'une centrifugeuse BECKMAN L 50 munie d'un rotor 30 (appareil commercialisé par BECKMANN) tournant à 10.000 t/mn pendant 15 mn, puis séchées à l'étuve sous vide à 40 ° C et conservées sous azote.

Les caractéristiques des particules sont conservées.

Les abréviations relatives au polymère de départ figurant dans les tableaux I et II ont la signification suivante :

PS : polystyrène
PS mag : polystyrène magnétique
PVC : polychlorure de vinyle
P(S/MAM) : copolymère styrène/méthacrylate de méthyle
ES : extrait sec

TABLEAU I

| EXEMPLE | 1 | 2 | 3 | 4 | 5 | 6 |
|---|---|---|---|---|---|---|
| LATEX - Polymère | PS | PS | PS | PS mag | PVC | P(S/MAM) |
| LATEX - Ø µm | 0,9 | 0,9 | 0,9 | 0,5-0,9 | 0,7 | 0,8 |
| LATEX - ES % | 33 | 33 | 33 | 33 | 37 | 30 |
| COMPOSE AMPHIPHILE - nature | CA-OH | CA-OH | CA-OH | CA-OH | CA-OH | CA-OH |
| COMPOSE AMPHIPHILE - g/100 g de polymère | 10 | 5 | 20 | 5 | 10 | 10 |
| COMPOSE AMPHIPHILE - moles /100 g de polymère | 5,35 | 2,675 | 11,70 | 2,675 | 5,35 | 5,35 |
| température °C | 70 | 70 | 70 | 70 | 80 | 90 |
| durée h | 2 | 2 | 2 | 2 | 2 | 2 |
| % implanté - théorie | 9,1 | 4,8 | 16,7 | 4,8 | 9,1 | 9,1 |
| % implanté - dosé | 6,4 | 3,1 | 12,7 | 2,9 | 5,8 | 7,1 |

TABLEAU II

| EXEMPLE | | 7 | 8 | 9 | 10 | 11 | 12 |
|---|---|---|---|---|---|---|---|
| LATEX | - Polymère | PS | PS | PS mag | PS | PS | PS |
| | - Ø µm | 0,9 | 0,9 | 0,5-0,9 | 0,9 | 0,8 | 0,9 |
| | - ES % | 33 | 33 | 33 | 33 | 33 | 33 |
| COMPOSE AMPHIPHILE | - nature | CASC | CA-SH | CASC | CA-COOH | CASC | CA-OH |
| | - g/100 g de polymère | 10 | 10 | 10 | 10 | 5 | 10 |
| | - moles /100 g de polymère | 4,94 | 2,55 | 4,94 | 4,95 | 2,47 | 5,35 |
| température °C | | 70 | 70 | 70 | 70 | 45 | 70 |
| durée h | | 2 | 2 | 2 | 2 | 2 | 2 |
| % implanté | - théorie | 9,1 | 9,1 | 9,1 | 9,1 | 4,8 | 9,1 |
| | - dosé | 5,3 | 4,8 | 3,1 | 6,2 | 4,3 | 6,4 |

## Revendications

1. Nouvelles particules de polymère caractérisées en ce qu'elles comportent, implantées à leur surface, des molécules d'un composé amphiphile de HLB supérieur ou égal à 10 et de masse moléculaire supérieure ou égale à 400 constitué d'une séquence hydrophile oligomère terminée par au moins un groupe ionogène ou réactif et d'une séquence hydrophobe, la quantité de molécules amphiphiles

implantées correspondant à $10^{-5}$ à $10^{-1}$ mole pour 100 g de polymère, le polymère constituant lesdites particules présentant une température de transition vitreuse Tg supérieure à environ 40 ° C, le diamètre desdites particules étant de l'ordre de 0,01 à 50 $\mu$m et l'implantation étant telle que le composé amphiphile pénètre par sa partie hydrophobe dans la couche périphérique de la particule et que ladite partie hydrophobe est emmêlée avec les chaînes de polymère, le composé amphiphile n'étant ainsi pas simplement adsorbé à la surface des particules.

**2.** Nouvelles particules selon la revendication 1 caractérisées en ce que le composé amphiphile présente un HLB inférieur à 20, la quantité de molécules amphiphiles implantées correspond à environ $10^{-4}$ à $10^{-2}$ mole pour 100 g de polymère, le polymère constituant les particules présente une Tg supérieure à environ 70 ° C et le diamètre des particules est de l'ordre de 0,01 à 15 $\mu$m.

**3.** Nouvelles particules selon la revendication 1 ou 2 caractérisées en ce que le polymère constituant les particules est un homopolymère ou un copolymère contenant des motifs dérivés des monomères vinylaromatiques, des alkylesters d'acides $\alpha$-$\beta$ insaturés, des esters d'acides carboxyliques insaturés, du chlorure de vinyle, du chlorure de vinylidène, des diènes, de ceux présentant des fonctions nitriles.

**4.** Nouvelles particules selon l'une quelconque des revendications précédentes caractérisées en ce que le composé amphiphile implanté en surface présente une séquence hydrophile oligomère polyoxyalkylénée contenant de 5 à 100 motifs oxyalkylénés en $C_2$-$C_3$, polycarboxyethylénée et/ou polyamidoéthylénée et/ou polycyanoéthylénée contenant de 4 à 50 motifs carboxyéthylénés et/ou amidoéthylénés et/ou cyanoéthylénés, ladite séquence hydrophile oligomère étant terminée par un groupe -OH, $-SO_3H$, -COOH, -CHO, $-ØCH_2Cl$, $-NH_2$, $-NR_2$, $NR_3$ (R étant un radical alkyle en $C_1$ - $C_2$), $-CONH_2$, $-NH-NH_2$, $-NH-NH-CO-NH_2$, -SH,

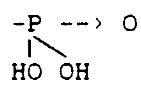

**5.** Nouvelles particules selon l'une quelconque des revendications précédentes caractérisées en ce qu'elles sont magnétisables.

**6.** Nouvelles dispersions aqueuse de particules de polymère caractérisées en ce qu'elles contiennent de 1 à 50 % de leur poids des nouvelles particules faisant l'objet de l'une quelconque des revendications 1 à 5.

**7.** Nouvelle dispersions selon la revendication 6 caractérisées en ce qu'elles contiennent de 5 à 30 % de leur poids de particules.

**8.** Procédé de préparation de dispersions aqueuses faisant l'objet de la revendication 6 caractérisé en ce qu'il comporte les étapes suivantes :

a) un composé amphiphile de HLB supérieur ou égal à 10 et de masse moléculaire supérieure ou égale à 400 est mis en contact avec un latex contenant de 1 à 50 % en poids de particules d'un polymère dont la température de transition vitreuse Tg est supérieure à environ 40 ° C, lesdites particules constituant le latex présentant un diamètre de l'ordre de 0,01 à 50 $\mu$m, la quantité dudit composé amphiphile mis en oeuvre correspondant à environ $10^{-4}$ à 1 mole pour 100 g de polymère, cette opération de mise en contact étant réalisée à une température comprise dans la zone de transition vitreuse dudit polymère, jusqu'à emmêlement des chaines de polymère et des séquences hydrophobes dudit composé amphiphile

b) élimination de tout composé amphiphile non fixé par implantation.

**9.** Procédé de préparation des nouvelles particules faisant l'objet de la revendication 1, caractérisé en ce qu'il comprend les étapes suivantes :

a) un composé amphiphile de HLB supérieur ou égal à 10 et de masse moléculaire supérieure ou égale à 400 est mis en contact avec un latex de particules d'un polymère dont la température de transition vitreuse Tg est supérieure à environ 40 ° C, lesdites particules constituant le latex

présentant un diamètre de l'ordre de 0,01 à 50 µm, la quantité dudit composé amphiphile mis en oeuvre correspondant à environ $10^{-4}$ à 1 mole pour 100 g de polymère, cette opération de mise en contact étant réalisée à une température comprise dans la zone de transition vitreuse dudit polymère, jusqu'à emmêlement des chaines de polymère et des séquences hydrophobes dudit composé amphiphile
b) élimination de tout composé amphiphile non fixé par implantation
c) séparation des particules ainsi obtenues.

**10.** Procédé selon la revendication 8 ou 9 caractérisé en ce que le composé amphiphile présente un HLB inférieur à 20.

**11.** Procédé selon l'une quelconque des revendications 8 à 10 caractérisé en ce que le composé amphiphile présente une séquence hydrophile oligomère polyoxyalkylénée contenant de 5 à 100 motifs oxyalkylénés en $C_2$-$C_3$, polycarboxylethylénée et/ou polyamidoéthylénée et/ou polycyanoéthylénée contenant de 4 à 50 motifs carboxyéthylénés et/ou amidoéthylénés et/ou cyanoéthylénés, ladite séquence hydrophile oligomère étant terminée par un groupe -OH, $-SO_3H$, -COOH, -CHO, $-ØCH_2Cl$, $-NH_2$, $-NR_2$, $NR_3$ (R étant un radical alkyle en $C_1$ - $C_2$), $-CONH_2$, $-NH-NH_2$, $-NH-NH-CO-NH_2$, -SH,

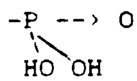

**12.** Procédé selon l'une quelconque des revendicatios 9 à 11 caractérisé en ce que le latex de particules comporte de 1 à 50 % en poids de particules de polymère.

**13.** Procédé selon l'une quelconque des revendications 8 à 12 caractérisé en ce que le latex de particules comporte de 5 à 30 % en poids de particules de polymère.

**14.** Procédé selon l'une quelconque des revendications 8 à 13 caractérisé en ce que lesdites particules présentent un diamètre de l'ordre de 0,01 à 15 µm.

**15.** Procédé selon l'une quelconque des revendications 8 à 14 caractérisé en ce que ledit polymère constituant les particules est un homopolymère ou un copolymère contenant des motifs dérivés des monomères vinylaromatiques, des alkylesters d'acides $\alpha$-$\beta$ insaturés, des esters d'acides carboxyliques insaturés, du chlorure de vinyle, du chlorure de vinylidène, des diènes, de ceux présentant des fonctions nitriles.

**16.** Procédé selon l'une quelconque des revendications 8 à15 caractérisé en ce que les particules dudit latex sont magnétisables.

**17.** Procédé selon l'une quelconque des revendications 8 à 16 caractérisé en ce que l'opération de mise en contact est réalisée en introduisant le composé amphiphile dans la phase aqueuse du latex à température ordinaire, puis en élevant la température jusqu'à une température comprise dans la zone de transition vitreuse du polymère constituant le latex.

**18.** Utilisation des particules faisant l'objet de l'une quelconque des revendications 1 à 5 en biologie.

**19.** Utilisation des dispersions de particules faisant l'objet de la revendication 6 ou 7 en biologie.

## Claims

**1.** Novel polymer particles, characterised in that they carry, implanted on their surface, molecules of an amphiphilic compound of HLB greater than or equal to 10 and of molecular weight greater than or equal to 400, consisting of a hydrophilic oligomer block terminated by at least one ion-forming or reactive group and a hydrophobic block, the quantity of amphiphilic molecules implanted corresponding to $10^{-5}$ to $10^{-1}$ mol per 100 g of polymer, the polymer constituting the said particles having a glass transition temperature Tg above about 40° C, the diameter of the said particles being of the order of 0.01 to 50

μm, and the implantation being such that the amphiphilic compound penetrates by its hydrophobic part into the peripheral layer of the particle and the said hydrophobic part is enmeshed with the polymer chains, the amphiphilic compound thus not being simply adsorbed on the surface of the particles.

**2.** Novel particles according to Claim 1, characterised in that the amphiphilic compound has an HLB less than 20, the quantity of amphiphilic molecules implanted corresponds to about $10^{-4}$ to $10^{-2}$ mol per 100 g of polymer, the polymer constituting the particles has a Tg above about 70 ° C and the diameter of the particles is of the order of 0.01 to 15 μm.

**3.** Novel particles according to Claim 1 or 2, characterised in that the polymer constituting the particles is a homopolymer or a copolymer containing units derived from vinylaromatic monomers, alkyl esters of α,β-unsaturated acids, esters of unsaturated carboxylic acids, vinyl chloride, vinylidene chloride, dienes or monomers possessing nitrile functional groups.

**4.** Novel particles according to any one of the preceding claims, characterised in that the surface-implanted amphiphilic compound has a hydrophilic oligomer polyoxyalkylene block containing from 5 to 100 $C_2$-$C_3$ oxyalkylene units or a hydrophilic oligomer polycarboxyethylene and/or polyamidoethylene and/or polycyanoethylene block containing from 4 to 50 carboxyethylene and/or amidoethylene and/or cyanoethylene units, the said hydrophilic oligomer block being terminated by an -OH, $-SO_3H$, -COOH, -CHO, $-OCH_2Cl$, $-NH_2$, $-NR_2$, $NR_3$ (R being a $C_1$-$C_2$-alkyl radical), $-CONH_2$, $-NH-NH_2$, $-NH-NH-CO-NH_2$, -SH, or

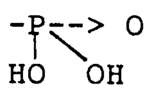

group.

**5.** Novel particles according to any of the preceding claims, characterised in that they are magnetisable.

**6.** Novel aqueous dispersions of polymer particles, characterised in that they contain from 1 to 50% by weight of the novel particles according to any of Claims 1 to 5.

**7.** Novel dispersions according to Claim 6, characterised in that they contain from 5 to 30% by weight of particles.

**8.** Process for the preparation of aqueous dispersions according to Claim 6, characterised in that it comprises the following stages:
a) an amphiphilic compound, with an HLB greater than or equal to 10 and a molecular weight greater than or equal to 400, is brought into contact with a latex containing from 1 to 50% by weight of particles of a polymer, whose glass transition temperature Tg is greater than about 40 ° C, the said particles constituting the latex having a diameter of the order of 0.01 to 50 μm and the amount of the said amphiphilic compound employed corresponding to about $10^{-4}$ to 1 mol per 100 g of polymer, this operation of bringing into contact being carried out at a temperature within the glass transition zone of the said polymer, until enmeshing of the polymer chains and of the hydrophobic blocks of the said amphiphilic compound has been achieved, and
b) removal of all the amphiphilic compound which has not been fixed by implantation.

**9.** Process for the preparation of the novel particles according to Claim 1, characterised in that it comprises the following stages:
a) an amphiphilic compound, with an HLB greater than or equal to 10 and a molecular weight greater than or equal to 400, is brought into contact with a latex of particles of a polymer, whose glass transition temperature Tg is greater than about 40 ° C, the said particles constituting the latex having a diameter of the order of 0.01 to 50 μm and the amount of the said amphiphilic compound employed corresponding to about $10^{-4}$ to 1 mol per 100 g of polymer, this operation of bringing into contact being carried out at a temperature within the glass transition zone of the said polymer, until enmeshing of the polymer chains and of the hydrophobic blocks of the said amphiphilic compound

has been achieved,
b) removal of all the amphiphilic compound which has not been fixed by implantation and
c) isolation of the particles thus obtained.

**10.** Process according to Claim 8 or 9, characterised in that the amphiphilic compound has an HLB of less than 20.

**11.** Process according to any of Claims 8 to 10, characterised in that the amphiphilic compound has a hydrophilic oligomer polyoxyalkylene block containing from 5 to 100 $C_2$-$C_3$ oxyalkylene units or a hydrophilic oligomer polycarboxyethylene and/or polyamidoethylene and/or polycyanoethylene block containing from 4 to 50 carboxyethylene and/or amidoethylene and/or cyanoethylene units, the said hydrophilic oligomer block being terminated by an -OH, $-SO_3H$, -COOH, -CHO, $-OCH_2Cl$, $-NH_2$, $-NR_3$ - (R being a $C_1$-$C_2$-alkyl radical), $-CONH_2$, $-NH-NH_2$, $-NH-NH-CO-NH_2$, -SH or

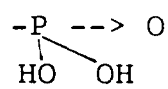

group.

**12.** Process according to any of Claims 9 to 11, characterised in that the latex of particles contains from 1 to 50% by weight of polymer particles.

**13.** Process according to any of Claims 8 to 12, characterised in that the latex of particles contains from 5 to 30% by weight of polymer particles.

**14.** Process according to any of Claims 8 to 13, characterised in that the said particles have a diameter of the order of 0.01 to 15 $\mu$m.

**15.** Process according to any of Claims 8 to 14, characterised in that the said polymer constituting the particles is a homopolymer or a copolymer containing units derived from vinylaromatic monomers, alkyl esters of $\alpha,\beta$-unsaturated acids, esters of unsaturated carboxylic acids, vinyl chloride, vinylidene chloride, dienes or those having nitrile functional groups.

**16.** Process according to any of Claims 8 to 15, characterised in that the particles of the said latex are magnetisable.

**17.** Process according to any of Claims 8 to 16, characterised in that the operation of bringing the components into contact is carried out by introducing the amphiphilic compound into the aqueous phase of the latex at ambient temperature and then raising the temperature to a temperature within the glass transition zone of the polymer constituting the latex.

**18.** Use of the particles as claimed in any of Claims 1 to 5 in biology.

**19.** Use of the dispersions of particles as claimed in Claim 6 or 7 in biology.

**Patentansprüche**

**1.** Neue Polymerteilchen, dadurch gekennzeichnet, daß sie an ihrer Oberfläche implantiert Moleküle einer amphiphilen Verbindung mit einem HLB-Wert ≧ 10 und einer Molekularmasse ≧ 400, bestehend aus einer durch mindestens eine ionogene oder reaktionsfähige Gruppe abgeschlossenen hydrophilen oligomeren Sequenz und aus einer hydrophoben Sequenz enthalten, wobei die Menge der implantierten amphiphilen Moleküle $10^{-5}$ bis $10^{-1}$ mol je 100 g Polymer entspricht, wobei das Polymer, aus dem die Teilchen bestehen, eine Glasübergangstemperatur Tg von über etwa 40 °C aufweist und der Teilchendurchmesser in der Größenordnung von 0,01 bis 50 $\mu$m liegt und wobei die Implantation so beschaffen ist, daß die amphiphile Verbindung mit ihrem hydrophoben Teil in die periphere Schicht des Teilchens eindringt und der hydrophobe Teil mit den Polymerketten verschlungen ist, so daß die

amphiphile Verbindung nicht nur einfach an der Oberfläche der Teilchen adsorbiert ist.

**2.** Neue Teilchen nach Anspruch 1, dadurch gekennzeichnet, daß die amphiphile Verbindung einen HLB-Wert unterhalb 20 aufweist, die Menge der implantierten amphiphilen Moleküle etwa $10^{-4}$ bis $10^{-2}$ mol je 100 g Polymer entspricht, das Polymer, aus dem die Teilchen bestehen, eine Tg über etwa 70 °C aufweist und der Teilchendurchmesser in der Größenordnung von 0,01 bis 15 $\mu$m liegt.

**3.** Neue Teilchen nach Anspruch 1 oder 2, dadurch gekennzeichnet, daß das Polymer, aus dem die Teilchen bestehen, ein Homopolymer oder ein Copolymer ist, welches Gruppen enthält, die von vinylaromatischen Monomeren, Alkylestern $\alpha$-$\beta$-ungesättigter Säuren, Estern ungesättigter Carbonsäuren, vinylchlorid, Vinylidenchlorid, Dienen und von Monomeren mit Nitrilfunktionen bzw. -gruppen abgeleitet sind.

**4.** Neue Teilchen nach einem der vorangehenden Ansprüche, dadurch gekennzeichnet, daß die an der Oberfläche implantierte amphiphile Verbindung eine hydrophile oligomere Polyoxyalkylen-Sequenz mit 5 bis 100 $C_2$-$C_3$-Oxyalkylengruppen, eine Polycarboxyethylen- und/oder Polyamidoethylen- und/oder Polycyanoethylen-Sequenz mit 4 bis 50 Carboxyethylen- und/oder Amidoethylen- und/oder Cyanoethylen-Gruppen aufweist, wobei die hydrophile oligomere Sequenz durch eine Gruppe -OH, $-SO_3H$, -COOH, -CHO, $-\Phi CH_2Cl$, $-NH_2$, $-NR_2$, $NR_3$ (R = $C_1$ - $C_2$-Alkylgruppe), $-CONH_2$, $-NH-NH_2$, $-NH-NH-CO-NH_2$, -SH,

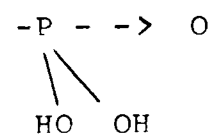

abgeschlossen ist.

**5.** Neue Teilchen nach einem der vorangehenden Ansprüche, dadurch gekennzeichnet, daß sie magnetisierbar sind.

**6.** Neue wäßrige Dispersionen von Polymerteilchen, dadurch gekennzeichnet, daß sie 1 bis 50 %, bezogen auf ihr Gewicht, neue Teilchen gemäß einem der Ansprüche 1 bis 5 enthalten.

**7.** Neue Dispersionen nach Anspruch 6, dadurch gekennzeichnet, daß sie 5 bis 30 %, bezogen auf ihr Gewicht, Teilchen enthalten.

**8.** Verfahren zur Herstellung von wäßrigen Dispersionen nach Anspruch 6, dadurch gekennzeichnet, daß es folgende Stufen umfaßt:

a) eine amphiphile Verbindung mit einem HLB-Wert ≧ 10 und einer Molekularmasse ≧ 400 wird mit einem Latex in Berührung gebracht, der 1 bis 50 Gew.-% Teilchen eines Polymeren enthält, dessen Glasübergangstemperatur Tg über etwa 40 °C liegt, wobei der Durchmesser der Teilchen, die den Latex ausmachen, im Bereich von 0,01 bis 50 $\mu$m liegt, wobei die Menge der eingesetzten amphiphilen Verbindung etwa $10^{-4}$ bis 1 mol je 100 g Polymer entspricht und wobei diese Maßnahme des Zusammenbringens bei einer Temperatur ausgeführt wird, die im Glasübergangsbereich des Polymeren liegt, bis die Ketten des Polymeren und die hydrophoben Sequenzen der amphiphilen Verbindung miteinander verschlungen sind,

b) Eliminierung der nicht durch Implantation fixierten amphiphilen Verbindung.

**9.** Verfahren zur Herstellung der neuen Teilchen nach Anspruch 1, dadurch gekennzeichnet, daß es folgende Stufen umfaßt:

a) eine amphiphile Verbindung mit einem HLB-Wert ≧ 10 und einer Molekularmasse ≧ 400 wird mit einem Latex von Teilchen eines Polymeren in Berührung gebracht, dessen Glasübergangstemperatur Tg über etwa 40 °C liegt, wobei der Durchmesser der Teilchen, die den Latex ausmachen, im Bereich von 0,01 bis 50 $\mu$m liegt, wobei die Menge an eingesetzter amphiphiler Verbindung etwa $10^{-4}$ bis 1 mol je 100 g Polymer entspricht und wobei diese Maßnahme des Zusammenbringens bei einer Temperatur, die im Glasübergangsbereich des Polymeren liegt, ausaeführt wird, bis die

Polymerketten und die hydrophoben Sequenzen der amphiphilen Verbindung verschlungen sind,
b) Entfernen der nicht durch Implantation fixierten amphiphilen Verbindung,
c) Abtrennen bzw. Isolieren der so erhaltenen Teilchen.

**10.** Verfahren nach Anspruch 8 oder 9, dadurch gekennzeichnet, daß die amphiphile Verbindung einen HLB-Wert unter 20 aufweist.

**11.** Verfahren nach einem der Ansprüche 8 bis 10, dadurch gekennzeichnet, daß die amphiphile Verbindung eine hydrophile oligomere Polyoxyalkylen Sequenz mit 5 bis 100 $C_2$-$C_3$-Oxyalkylengruppen, eine Polycarboxyethylen- und/oder Polyamidoethylen- und/oder Polycyanoethylen-Sequenz mit 4 bis 50 Carboxyethylen- und/oder Amidoethylen- und/oder Cyanoethylen-Gruppen aufweist, wobei die hydrophile oligomere Sequenz durch eine Gruppe -OH, $-SO_3H$, -COOH, -CHO, $-\Phi CH_2Cl$, $-NH_2$, $-NR_2$, $NR_3$ (R = $C_1$ - $C_2$-Alkylgruppe), $-CONH_2$, $-NH-NH_2$, $-NH-NH-CO-NH_2$, -SH,

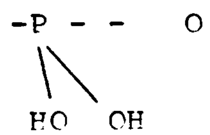

abgeschlossen ist.

**12.** Verfahren nach einem der Ansprüche 9 bis 11, dadurch gekennzeichnet, daß der Teilchenlatex 1 bis 50 Gew.-% Polymerteilchen enthält.

**13.** Verfahren nach einem der Ansprüche 8 bis 12, dadurch gekennzeichnet, daß der Teilchenlatex 5 bis 30 Gew.-% Polymerteilchen enthält.

**14.** Verfahren nach einem der Ansprüche 8 bis 13, dadurch gekennzeichnet, daß die Teilchen einen Durchmesser im Bereich von 0,01 bis 15 $\mu$m aufweisen.

**15.** Verfahren nach einem der Ansprüche 8 bis 14, dadurch gekennzeichnet, daß das Polymer, aus dem die Teilchen bestehen, ein Homopolymer oder ein Copolymer ist, welches Gruppen enthält, die von vinylaromatischen Monomeren, Alkylestern $\alpha$-$\beta$-ungesättigter Säuren, Estern ungesättigter Carbonsäuren, Vinylchlorid, Vinylidenchlorid, Dienen und von Monomeren mit Nitrilfunktionen bzw. -gruppen abgeleitet sind.

**16.** Verfahren nach einem der Ansprüche 8 bis 15, dadurch gekennzeichnet, daß die Latexteilchen magnetisierbar sind.

**17.** Verfahren nach einem der Ansprüche 8 bis 16, dadurch gekennzeichnet, daß die Maßnahme des Zusammenbringens ausaeführt wird, indem die amphiphile Verbindung bei gewöhnlicher Temperatur in die wäßrige Phase des Latex eingebracht und dann die Temperatur auf eine Temperatur im Glasübergangsbereich des Polymeren des Latex erhöht wird.

**18.** Verwendung der Teilchen nach einem der Ansprüche 1 bis 5 in der Biologie.

**19.** Verwendung der Dispersionen von Teilchen nach einem der Ansprüche 6 oder 7 in der Biologie.